# EUROPEAN PATENT APPLICATION

(11) **EP 3 613 848 A1**
(43) Date of publication of application: **26.02.2020**
(21) Application number: 18787555.4
(22) Date of filing: 11.04.2018
(51) Int. Cl.: C12N 5/0793, C12N 1/00, C12N 5/10

(54) **METHOD FOR PRODUCING DOPAMINERGIC NEURONS**

(30) Priority: 19.04.2017 JP 2017082600
(71) Applicant: National University Corporation Nagoya University, Nagoya-shi, Aichi 464-8601 (JP)
(72) Inventor: OZAKI Norio, Nagoya-shi Aichi 464-8601 (JP); ARIOKA Yuko, Nagoya-shi Aichi 464-8601 (JP); MORI Daisuke, Nagoya-shi Aichi 464-8601 (JP); KUSHIMA Itaru, Nagoya-shi Aichi 464-8601 (JP)
(74) Representative: Witthoff Jaekel Steinecke Patentanwälte PartG mbB
(86) International application number: PCT/JP2018/015304
(87) International publication number: WO 2018/193949

(57) **Abstract**

An object of the present invention is to provide a method for preparing dopamine neurons efficiently in a short period of time. The dopamine neuron is prepared by step (1) of culturing pluripotent stem cells in the presence of a TGF-β family inhibitor, a GSK3β inhibitor, and a BMP inhibitor; step (2) of suspension-culturing the cells obtained in step (1) in the presence of a TGF-β family inhibitor, a GSK3β inhibitor, FGF8, and a hedgehog signal agonist and under normal oxygen partial pressure to form a neurosphere; and step (3) of collecting the neurosphere to induce differentiation of the cells into dopamine neurons.

## Description

### [Technical Field]

The present invention relates to a method for preparing dopamine neurons (dopaminergic neurons) and use thereof. The present application claims priority based on Japanese Patent Application No. 2017-082600 filed on April 19, 2017, the entire contents of which are incorporated herein by reference.

### [Background Art]

Pluripotent stem cells represented by induced pluripotent stem cells (iPS cells) are expected to be applied in various fields such as drug development, regenerative medicine, and basic research. When pluripotent stem cells are cultured under appropriate conditions, they differentiate along specific cell lineages. Also with respect to cells that construct the nervous system, attempts have been made to prepare various types of neurons by taking advantage of this characteristic. For example, a method of culturing pluripotent stem cells under low oxygen partial pressure and inducing differentiation thereof into various neurons and glial cells has been proposed (PTL 1, and NPLs 1 and 2). There is also a report that high-quality dopamine neurons can be induced by using a medium containing cAMP and a MEK inhibitor (PTL 2). In addition to these reports, there are many reports on methods for inducing differentiation of dopamine neurons or their precursor cells (for example, NPLs 3 to 6).

### [Citation List]

### [Patent Literatures]

[PTL 1] WO 2013/187416 A1
[PTL 2] WO 2015/020234 A1

### [Non Patent Literatures]

[NPL 1] Stem Cell Reports 2016 Mar8; 6 (3): 422-35
[NPL 2] Molecular Brain (2016) 9:88
[NPL 3] PLoS One. 2014 Feb 21; 9 (2): e87388
[NPL 4] Biochim Biophys Acta. 2015 Sep; 1850 (9): 1669-75
[NPL 5] Biochim Biophys Acta. 2015 Sep; 1850 (1): 22-32
[NPL 6] Nat Commun. 2016 Oct 14; 7: 13097

### [Summary of Invention]

### [Technical Problem]

Although there are various attempts to prepare neurons (especially, dopamine neurons) from pluripotent stem cells, there are still many problems to be overcome. For example, in the method disclosed in PTL 1 presented above, the use of the special oxygen condition, i.e., under low oxygen partial pressure, provides a major obstacle. In addition, there is room for improvement in the differentiation efficiency (differentiation specificity) into dopamine neurons. Furthermore, the period required to obtain the dopamine neurons is long. In order to advance clinical application, it is desirable to create a method or conditions for obtaining dopamine neurons efficiently in a short period of time.

### [Solution to Problem]

In order to solve the above problems, the present inventors have advanced research for creating a novel method for preparing dopamine neurons. In particular, the present inventors have made studies while considering that a dedicated device is required for culturing pluripotent stem cells under low oxygen partial pressure, which would provide a major obstacle in putting such a method into practical use. As a result, the present inventors have succeeded in establishing a novel method (protocol) that can induce differentiation of pluripotent stem cells into dopamine neurons efficiently and in a short period of time even without using a special oxygen condition, i.e., under low oxygen partial pressure. In addition, it has been demonstrated that the dopamine neurons prepared by this method are useful for various assays. That is, a method for preparing dopamine neurons excellent in versatility and practicality has been successfully established. The fact that the differentiation of pluripotent stem cells into dopamine neurons can be induced specifically/efficiently and, besides, in a short period of time even without adopting a special oxygen condition, i.e., under low oxygen partial pressure, greatly advances transplantation medicine and drug development utilizing pluripotent stem cells, and its significance is extremely large.

The following inventions are based mainly on the above results and considerations.
[1] A method for preparing dopamine neurons, including the following steps (1) to (3):
   (1) culturing pluripotent stem cells in the presence of a TGF-β family inhibitor, a GSK3β inhibitor, and a BMP inhibitor;
   (2) suspension-culturing the cells obtained in step (1) in the presence of a TGF-β family inhibitor, a GSK3β inhibitor, FGF8, and a hedgehog signal agonist and under normal oxygen partial pressure to form a neurosphere; and
   (3) collecting the neurosphere to induce differentiation of the cells into dopamine neurons.
[2] The preparation method according to [1], in which the pluripotent stem cells are induced pluripotent stem cells.
[3] The preparation method according to [1] or [2], in which the pluripotent stem cells are human cells.
[4] The preparation method according to any one of [1] to [3], in which the TGF-β family inhibitor is 4-[4-(1,3-benzodioxol-5-yl)-5-(2-pyridinyl)-1H-imidazol-2-yl]-benzamide or a hydrate thereof.
[5] The preparation method according to any one of [1] to [4], in which the GSK3β inhibitor is 6-[[2-[[4-(2,4-dichlorophenyl)-5-(4-methyl-1H-imidazol-2-yl)-2-pyrimidinyl]amino] ethyl] amino] nicotinonitrile.
[6] The preparation method according to any one of [1] to [5], in which the BMP inhibitor is 6-[4-(2-piperidin-1-ylethoxy)phenyl]-3-pyridin-4-ylpyrazolo[1,5-a]pyrimidine.
[7] The preparation method according to any one of [1] to [6], in which the hedgehog signal agonist is 9-cyclohexyl-N-[4-(4-morpholinyl)phenyl]-2-(1-naphthalenyloxy)-9H-purin-6-amine.
[8] The preparation method according to any one of [1] to [7], in which induction of differentiation into unnecessary cells such as glial cells is suppressed by suspension-culturing under the normal oxygen partial pressure in step (2).
[9] The preparation method according to any one of [1] to [8], in which the number of passages in step (2) is 0 or 1.
[10] The preparation method according to [9], in which promotion of unintended differentiation induction is avoided due to a small number of passages.
[11] The preparation method according to any one of [1] to [10], in which the culture period in step (1) is 4 days or longer.
[12] The preparation method according to any one of [1] to [11], in which all of steps (1) to (3) are carried out under the normal oxygen partial pressure.
[13] The preparation method according to any one of [1] to [12], in which the normal oxygen partial pressure is a condition where the oxygen concentration is 18% to 22%.
[14] The preparation method according to any one of [1] to [13], in which the culture in step (2) is performed under conditions where LIF, bFGF, and a ROCK inhibitor are further present.
[15] The preparation method according to any one of [1] to [14], in which the culture period in step (2) is 7 days to 21 days.
[16] The preparation method according to any one of [1] to [15], in which step (3) includes adherent culture in the presence of a γ-secretase inhibitor, a neurotrophic factor, ascorbic acid, TGF-β3 and cAMP or a cAMP analog.
[17] The preparation method according to [16], in which the γ-secretase inhibitor is N-[N-(3,5-difluorophenacetyl-L-alanyl)]-S-phenylglycine t-butyl ester, in which the neurotrophic factor is a brain-derived neurotrophic factor (BDNF) and a glial cell-derived neurotrophic factor (GDNF), and in which the cAMP analog is diptyryl cAMP.
[18] The preparation method according to [16] or [17], in which the culture period in step (3) is 5 days or longer.
[19] A dopamine neuron obtained by the preparation method according to any one of [1] to [18].
[20] An *in vitro* assay using the dopamine neuron according to [19].

### [Brief Description of Drawings]

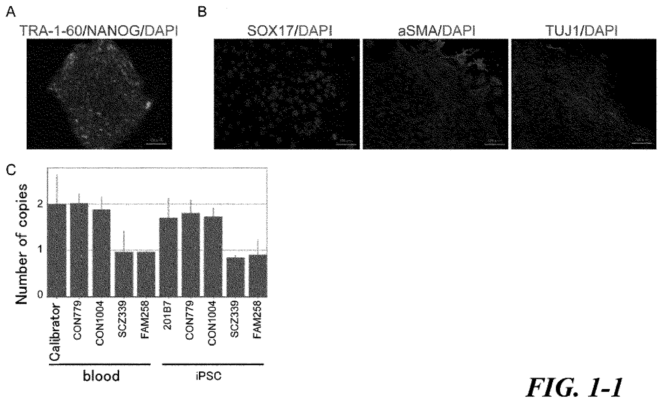

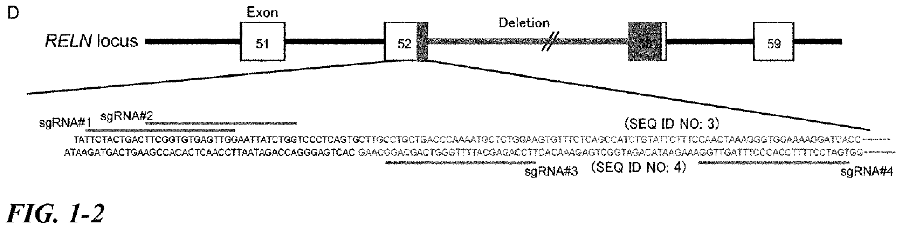

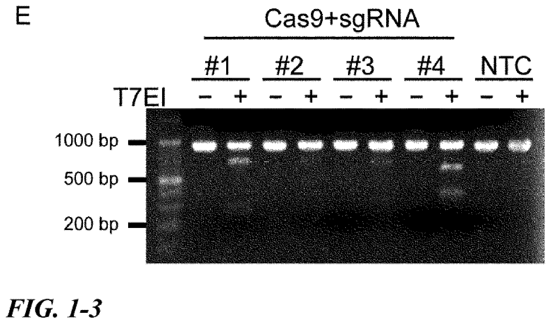

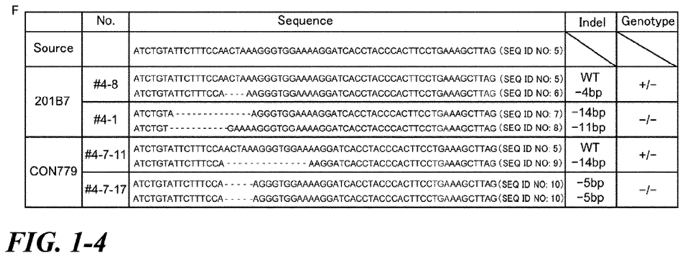

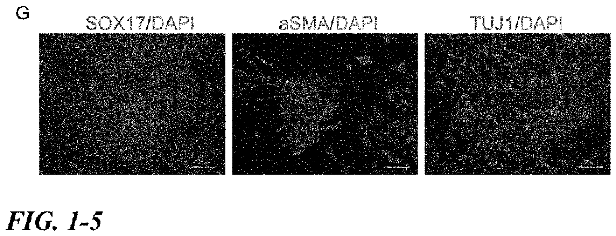

[Fig. 1-1] Establishment of RELN-deleted iPS cells. (A) An image of immunostaining for NANOG and TRA-1-60 in RELN-deleted iPS cells. (B) Confirmation of capacity to differentiate into three germ layers *in vitro.* (C) Confirmation of RELN deletion in RELN-deleted iPS cell genome (comparison between the blood genome and the iPS cell genome).
[Fig. 1-2] Continuation of Fig. 1. (D) Target site of CRISPR-sgRNA used.
[Fig. 1-3] Continuation of Fig. 1. (E) Evaluation of the CRISPR/sgRNA activity by a T7EI assay.
[Fig. 1-4] Continuation of Fig. 1. (F) A list of RELN-deleted isogenic lines obtained using CRISPR-sgRNA #4.
[Fig. 1-5] Continuation of Fig. 1. (G) Confirmation of capacity to differentiate into three germ layers of the RELN-deleted isogenic lines. 201B7-derived #4-1 (+/- heterozygous deletion).
[Fig. 2] Reelin expression decreases in neurons with RELN deletion. (A) Analysis of expression of RELN mRNA using healthy control iPS cell-derived neurospheres (Day 21) and dopamine neurons (Day 28). (B) Comparison of expression of RELN mRNA in the neurospheres (Day 21). (C) Comparison of expression of RELN mRNA in the dopamine neurons (Day 28). (D) Results of immunostaining with TH and Reelin in the dopamine neurons (Day 21) of 201B7, heterozygous deletion Ig201B7 (+/-) and homozygous deletion Ig201B7 (-/-).
[Fig. 3-1] Abnormal dopamine production in dopamine neurons derived from congenital RELN-deleted parent-child iPS cells. (A) Images of immunostaining for TH and Tuj1 in early dopamine neurons (Day 23). (B) TH-positive rate in Tuj1-positive cells of each group. (C) Comprehensive gene expression comparative analysis using neurospheres in healthy controls and RELN-deleted parent and child.
[Fig. 3-2] Continuation of Fig. 3. (D) Analysis of expression of COMT using quantitative PCR. (E) Measurement of dopamine concentration using the culture supernatant on Day 28.
[Fig. 4-1] Abnormal migration direction of neurons due to RELN deletion. (A) Real-time imaging analysis of neuronal migration. Left: phase contrast microscope image, right: cell tracking results.
[Fig. 4-2] Continuation of Fig. 4. (B) Total movement distance (a) for 4 hours. (C) Distance (b) between two points, i.e., start point and end point of shooting. (D) Ratio (b/a) × 100.
[Fig. 4-3] Continuation of Fig. 4. (E) Schematic diagram of cell migration angle analysis. (F) Left: Cell angle analysis results for one representative cell. Right: Summary of angle analysis results for 10 cells. (G) Comparison of migration angle.
[Fig. 5] Dopamine production ability of dopamine neurons prepared by a novel protocol. Healthy iPS cells were differentiated into dopamine neurons to measure the concentration of dopamine in the culture supernatants on Day 28 and Day 42 of culture. Day 28: 7 days after the start of differentiation induction from neurospheres, Day 42: 21 days after the start of differentiation induction from neurospheres, and NTC: value for a medium itself (without cells). An increase in dopamine amount over time can be confirmed.
[Fig. 6] Confirmation of dopamine neurons prepared by the novel protocol. Healthy iPS cells were differentiated into dopamine neurons, and fixed and immunostained on Day 28. Left: midbrain marker (FOXA2), center: dopamine neuron marker (TH), and right: Merge. Since double-positive cells exhibiting are observed, it can be seen that the iPS cells have been differentiated into midbrain dopamine neurons.

### [Description of Embodiments]

### 1. Method for preparing dopamine neurons

The present invention relates to a method for preparing dopamine neurons from pluripotent stem cells (hereinafter also referred to as "the preparation method of the present invention"). According to the present invention, cells exhibiting characteristics similar to those of dopamine neurons constituting the central nerve of a living body can be obtained. Dopamine neurons are useful as therapeutic agents or transplant materials (cells or tissues for transplantation medicine) for neurological diseases (for example, mental disorders and neurodegenerative diseases). They are also useful as tools for developing drugs (therapeutic agents and preventive agents) for neurological diseases and for studying the mechanisms for onset and progression of neurological diseases. The preparation method of the present invention having excellent versatility makes it possible to easily and inexpensively prepare dopamine neurons having such high usefulness. Moreover, according to the preparation method of the present invention, dopamine neurons can be efficiently obtained in a short period of time.

In the preparation method of the present invention, the following the steps (1) to (3) are performed:
(1) culturing pluripotent stem cells in the presence of a TGF-β family inhibitor, a GSK3β inhibitor, and a BMP inhibitor;
(2) suspension-culturing the cells obtained in step (1) in the presence of a TGF-β family inhibitor, a GSK3β inhibitor, FGF8, and a hedgehog signal agonist and under normal oxygen partial pressure to form a neurosphere; and
(3) collecting the neurosphere to induce differentiation of the cells into dopamine neurons.

### Step (1)

In step (1), pluripotent stem cells are used. The "pluripotent stem cell" refers to a cell having both the potential for differentiating into all cells constituting the body (pluripotency), and the potential for producing daughter cells having the same differentiation potency via cell division (self-replication competence). The pluripotency can be evaluated by transplanting cells of an evaluation subject into a nude mouse, and testing the presence or absence of formation of teratoma containing each cell of the three germ layers (ectoderm, mesoderm, and endoderm).

Examples of the pluripotent stem cells include embryonic stem cells (ES cells), embryonic germ cells (EG cells), and induced pluripotent stem cells (iPS cells), but the cells are not limited thereto as long as they have both the pluripotency and the self-replication competence. ES cells or iPS cells are preferably used. More preferably, iPS cells are used. Preferably, pluripotent stem cells are cells of mammals (for example, primates such as humans or chimpanzees, and rodents such as mice or rats), and particularly preferably, pluripotent stem cells are human cells. Therefore, in a most preferable embodiment, human iPS cells are used as pluripotent stem cells.

ES cells can be established by culturing, for example, a pre-implantation early embryo, an inner cell mass that constitutes the early embryo, a single blastomere, and the like (Manipulating the Mouse Embryo A Laboratory Manual, Second Edition, Cold Spring Harbor Laboratory Press (1994); Thomson, JA et al., Science, 282, 1145-1147 (1998)). As the early embryo, an early embryo prepared by nuclear-transplanting the nucleus of a somatic cell may be used (Wilmut et al. (Nature, 385, 810 (1997)), Cibelli et al. (Science, 280, 1256 (Nature, 394, 369 (1998)), Akira IRITANI et al. (Tanpakushitsu Kakusan Koso, 44, 892 (1999)), Baguisi et al. (Nature Biotechnology, 17, 456 (1999)), Wakayama et al. (Nature, 394, 369 (1998); Nature Genetics, 22, 127 (1999); Proc. Natl. Acad. Sci. USA, 96, 14984 (1999)), Rideout III et al. (Nature Genetics, 24, 109 (2000), Tachibana et al. Human Embryonic Stem Cells Derived by Somatic Cell Nuclear Transfer, Cell (2013) in press). As an early embryo, a parthenogenetic embryo may also be used: Kim et al. (Science, 315, 482-486 (2007)), Nakajima et al. (Stem Cells, 25, 983-985 (2007)), Kim et al. (Cell Stem Cell, 1, 346-352 (2007)), Revazova et al. (Cloning Stem Cells, 9, 432-449 (2007)), Revazova et al. (Cloning Stem Cells, 10, 11-24 (2008)). In addition to the above-mentioned papers, Stregkchenko N., et al. Reprod Biomed Online. 9: 623-629, 2004; Klimanskaya I., et al. Nature 444: 481-485, 2006; Chung Y., et al. Cell Stem Cell 2: 113-117, 2008; Zhang X., et al. Stem Cells 24: 2669-2676, 2006; Wassarman, P.M. et al. Methods in Enzymology, Vol. 365, 2003, etc. may also be referred to, as for the preparation of ES cells. Fused ES cells obtained by cell fusion of ES cells and somatic cells are also included in the embryonic stem cells used for the method of the present invention.

Some ES cells are available from preservation institutes or commercially available. For example, human ES cells are available from the Institute for Frontier Medical Sciences, Kyoto University (for example, KhES-1, KhES-2, and KhES-3), WiCell Research Institute, ESI BIO, and the like.

EG cells can be established by culturing primordial germ cells in the presence of LIF, bFGF, SCF, and the like (Matsui et al., Cell, 70, 841-847 (1992), Shamblott et al., Proc. Natl. Acad. Sci. USA, 95 (23), 13726-13731 (1998), Turnpenny et al., Stem Cells, 21 (5), 598-609, (2003)).

The "induced pluripotent stem cell (iPS cell)" refers to a cell having pluripotency and self-replication competence, produced by reprogramming somatic cells (e.g., fibroblasts, skin cells, and lymphocytes), for example, through the introduction of initializing factors. iPS cells show characteristics similar to those of ES cells. Somatic cells used in the preparation of iPS cells are not particularly limited, and may be differentiated somatic cells or undifferentiated stem cells. iPS cells can be prepared by various methods reported so far. The application of iPS cell preparation methods which will be developed in the future is also contemplated. Examples of the cells available for preparing iPS cells (i.e. cells from which iPS cells are derived) include lymphocytes (T cells, B cells), fibroblasts, epithelial cells, endothelial cells, mucosal epithelial cells, mesenchymal cells, hematopoietic stem cells, adipose stem cells, dental pulp stem cells and neural stem cells.

The most fundamental technique of iPS cell preparation methods is to introduce four factors of Oct3/4, Sox2, Klf4, and c-Myc, which are transcription factors, into cells by using virus (Takahashi K, Yamanaka S: Cell 126 (4), 663-676, 2006; Takahashi, K, et al.: Cell 131 (5), 861-72, 2007). The establishment of human iPS cells by introduction of four factors of Oct4, Sox2, Lin28, and Nonog has been reported (Yu J, et al.: Science 318 (5858), 1917-1920, 2007). The establishment of iPS cells by introduction of three factors other than c-Myc (Nakagawa M, et al.: Nat. Biotechnol. 26 (1), 101-106, 2008), two factors of Oct3/4 and Klf4 (Kim JB, et al.: Nature 454 (7204), 646-650, 2008), or only Oct3/4 (Kim JB, et al.: Cell 136 (3), 411-419, 2009) has also been reported. Also, a technique for introducing a protein, which is an expression product of a gene, into cells (Zhou H, Wu S, Joo JY, et al.: Cell Stem Cell 4, 381-384, 2009; Kim D, Kim CH, Moon JI, et al: Cell Stem Cell 4, 472-476, 2009) has also been reported. On the other hand, it has been reported that it is possible to improve the preparation efficiency and reduce the factors to be introduced, by using, for example, an inhibitor BIX-01294 against histone methyltransferase G9a, a histone deacetylase inhibitor valproic acid (VPA), or BayK8644 (Huangfu D, et al.: Nat. Biotechnol. 26 (7), 795-797, 2008; Huangfu D, et al.: Nat. Biotechnol. 26 (11), 1269-1275, 2008; Silva J, et al.: PLoS. Biol. 6 (10), e 253, 2008). Studies have also been advanced on gene transfer methods, and techniques utilizing not only retroviruses, but also lentiviruses (Yu J, et al.: Science 318 (5858), 1917-1920, 2007), adenoviruses (Stadtfeld M, et al.: Science 322 (5903), 945-949, 2008), plasmids (Okita K, et al.: Science 322 (5903), 949-953, 2008), transposon vectors (Woltjen K, Michael IP, Mohseni P, et al.: Nature 458, 766-770, 2009; Kaji K, Norrby K, Pac a A, et al.: Nature 458, 771-775, 2009; Yusa K, Rad R, Takeda J, et al.: Nat Methods 6, 363-369, 2009), or episomal vectors (Yu J, Hu K, Smuga-Otto K, Tian S, et al: Science 324, 797-801, 2009) for gene transfer have been developed.

Cells in which transformation into iPS cells, i.e., initialization (reprogramming) has occurred can be selected by using, as an indicator, the expression of pluripotent stem cell markers (undifferentiated markers) such as Fbxo15, Nanog, Oct/4, Fgf-4, Esg-1, and Cript.

iPS cells can also be provided from, for example, the National University Corporation, Kyoto University or the Independent Administrative Institution, RIKEN BioResource Center.

Pluripotent stem cells can be maintained in vitro by known methods. For example, when it is desired to provide highly safe cells (e.g. in a case where clinical application is considered), pluripotent stem cells are preferably maintained by serum-free culture using a serum alternative or by feeder-free cell culture. If a serum is used (or used in combination), autologous serum (i.e., recipient's serum) is preferably used. A serum alternative can be prepared by known methods (see, for example, WO 98/30679). Commercially available serum alternatives can also be used. Examples of the commercially available serum alternatives include KSR (manufactured by Invitrogen), Chemically-defined Lipid concentrated (manufactured by Gibco), and Glutamax (manufactured by Gibco).

In step (1), the pluripotent stem cells prepared as described above are cultured in the presence of a TGF-β family inhibitor, a GSK3β inhibitor, and a BMP inhibitor. That is, the pluripotent stem cells are cultured using a medium to which a TGF-β family inhibitor, a GSK3β inhibitor, and a BMP inhibitor are added. Step (1) aims to enhance the neuronal differentiation potency of the pluripotent stem cells.

The medium to be used can be prepared using a medium used for culturing mammalian cells as a basal medium. As the basal medium, for example, a BME medium, a BGJb medium, a CMRL 1066 medium, a Glasgow MEM medium, an Improved MEM Zinc Option medium, an IMDM medium, a Medium 199 medium, an Eagle MEM medium, an αMEM medium, a DMEM medium, a Ham's medium, a Ham's F-12 medium, a RPMI 1640 medium, a Fischer's medium, a Neurobasal medium, and a mixed medium thereof can be used, and the basal medium is not particularly limited as long as the it can be used for culturing mammalian cells. In one embodiment, a mixed medium of IMDM medium and Ham's F-12 medium is used. The mixing ratio is, for example, IMDM: Ham's F-12 = 0.8 to 1.2:1.2 to 0.8 in a volume ratio.

A TGF-β family inhibitor, a GSK3β inhibitor, and a BMP inhibitor are added to the medium. The TGF-β family inhibitor is a substance that inhibits TGF-β signaling through binding between TGF-β and a TGF-β receptor. The TGF-β inhibitor includes proteinaceous inhibitors and small molecule inhibitors. Examples of such proteinaceous inhibitors are anti-TGF-β neutralizing antibodies and anti-TGF-β receptor neutralizing antibodies. Examples of such small molecule inhibitors are SB431542 (4-[4-(1,3-benzodioxol-5-yl)-5-(2-pyridinyl)-1H-imidazol-2-yl]-benzamide or its hydrate), SB202190 (4-(4-fluorophenyl)-2-(4-hydroxyphenyl)-5-(4-pyridyl)-1H-imidazole), SB505124 (GlaxoSmithKline), NPC30345, SD093, SD908, SD208 (Scios), LY2109761, LY364947, and LY580276 (Lilly Research Laboratories). Preferably, SB431542 is used. The concentration of the TGF-β family inhibitor (amount to be added to the medium) is not particularly limited as long as the purpose of enhancing the neuronal differentiation potency of the pluripotent stem cells is achieved. When SB431542 is taken as an example, the concentration thereof is, for example, 0.5 µM to 20 µM, preferably 1 µM to 10 µM. The optimum concentration can be set through preliminary experiments. Instead of keeping the TGF-β family inhibitor concentration constant throughout the entire culture period, changes in TGF-β family inhibitor concentration, e.g., a stepwise increase in TGF-β family inhibitor concentration, may be provided.

Examples of usable GSK3β inhibitors include CHIR99021 (6-[[2-[[4-(2,4-dichlorophenyl)-5-(4-methyl-1H-imidazol-2-yl)-2-pyrimidinyl]amino] ethyl] amino]nicotinonitrile), SB-415286(3-[(3-chloro-4-hydroxyphenyl)amino]-4-(2-nitrophenyl)-1H-pyrrole-2,5-dione), SB-2167, Indirubin-3'-Monoxime, Kenpaullone, and BIO (6-bromoindirubin-3'-oxime). Preferably, CHIR99021 is used. The concentration of the GSK3β inhibitor (amount to be added to the medium) is not particularly limited as long as the purpose of enhancing the neuronal differentiation potency of the pluripotent stem cells is achieved. When CHIR99021 is taken as an example, the concentration thereof is, for example, 0.5 µM to 20 µM, preferably 1 µM to 10 µM. The optimum concentration can be set through preliminary experiments. Instead of keeping the GSK3β inhibitor concentration constant throughout the entire culture period, changes in GSK3β inhibitor concentration, e.g., a stepwise increase in GSK3β inhibitor concentration, may be provided.

The BMP inhibitor is a substance that inhibits BMP signaling through binding between BMP (bone morphogenetic protein) and a BMP receptor (type I or type II). The BMP inhibitor includes proteinaceous inhibitors and small molecule inhibitors. Examples of such proteinaceous inhibitors include natural inhibitors such as Noggin, chordin and follistatin. Examples of such small molecule inhibitors include Dorsomorphin (6-[4-(2-piperidin-1-ylethoxy)phenyl]-3-pyridin-4-ylpyrazolo[1,5-a]pyrimidine) and its derivatives, LDN-193189 (4-(6-(4-piperazin-1-yl)phenyl) pyrazolo[1,5-a]pyrimidin-3-yl) quinoline) and its derivatives. These compounds are commercially available (e.g., available from Sigma-Aldrich and Stemgent) and are readily available. Preferably, Dorsomorphin is used. The concentration of the BMP inhibitor (amount to be added to the medium) is not particularly limited as long as the purpose of enhancing the neuronal differentiation potency of the pluripotent stem cells is achieved. When Dorsomorphin is taken as an example, the concentration thereof is, for example, 0.5 µM to 20 µM, preferably 1 µM to 10 µM. The optimum concentration can be set through preliminary experiments. Instead of keeping the BMP inhibitor concentration constant throughout the entire culture period, changes in BMP inhibitor concentration, e.g., a stepwise increase in BMP inhibitor concentration, may be provided.

As necessary, the medium can contain other components. Examples of the components to be added include insulin, an iron source (e.g., transferrin), a mineral (e.g., sodium selenate), a saccharide (e.g., glucose), an organic acid (e.g., pyruvic acid or lactic acid), a serum protein (e.g., albumin), an amino acid (e.g., L-glutamine), a reducing agent (e.g., 2-mercaptoethanol), a vitamin (e.g., ascorbic acid or d-biotin), an antibiotic (e.g., streptomycin, penicillin or gentamicin), and a buffer (e.g., HEPES).

Pluripotent stem cells are usually subjected to adherent culture. The adherent culture is in contrast to suspension culture, and is typically two-dimensional culture (plane culture) under adherent conditions. However, Matrigel™ (BD) or the like may be used for three-dimensional culture. For example, dishes, Petri dishes, tissue culture dishes, multi-dishes, microplates, microwell plates, multi-plates, multi-well plates, chamber slides, laboratory dishes, and the like can be used for adherent culture. In order to enhance the adhesiveness of the cells to the culture surface, it is preferable to use a culture vessel coated with Matrigel™ (BD), poly-D-lysine, poly-L-lysine, collagen, gelatin, laminin, heparan sulfate proteoglycan, entactin, or a combination of two or more thereof.

The pluripotent stem cells can be cultured under either condition, i.e., either in the presence or absence of feeder cells. When it is desired to provide highly safe cells, e.g., when clinical application is taken into consideration, the pluripotent stem cells may be cultured in the absence of feeder cells (feeder cell-free culture). Examples of the feeder cells are MEFs (mouse embryonic fibroblasts), STO cells (mouse embryonic fibroblast cell line), and SNL cells (subclones of the STO cells).

Other culture conditions such as culture temperature, CO₂ concentration, and O₂ concentration can be set as appropriate. The culture temperature is, for example, about 30 to 40°C, preferably about 37°C. The CO₂ concentration is, for example, about 1 to 10%, preferably about 5%. Moreover, culture may be performed under normal oxygen partial pressure. The oxygen concentration in the case of the condition "under normal oxygen partial pressure" is typically about 18% to about 22%, though it may vary depending on other conditions (humidity, CO₂ concentration, and the like). The details of the condition "under normal oxygen partial pressure" will be described later.

The period (culture period) of step (1) is 4 days or longer, specifically, for example, 4 days to 20 days, preferably 6 days to 14 days. If the culture period is too short, the neurosphere formation ability will be reduced. On the other hand, if the culture period is excessively long, one of the effects of the present invention, that is, efficient preparation of dopamine neurons, can be impaired.

The cells may be subjected to subculture as necessary. For example, the cells are collected at a stage where they are brought in a subconfluent or confluent state, a part of the cells is seeded in another culture vessel, and culture is continued. A cell dissociation solution or the like may be used for cell collection. As the cell dissociation solution, for example, proteases such as EDTA-trypsin, collagenase IV, and metalloprotease can be used alone or in an appropriate combination. Cell dissociation solutions with low cell toxicity are preferred. As such cell dissociation solutions, commercially available products such as DISPASE (EIDIA Co., Ltd.), TrypLE (Invitrogen), and Accutase (MILLIPORE) are available. The collected cells may be subjected to subculture after treatment with a cell strainer or the like so as to arrive at a dispersed (discrete) state.

As a result of step (1), the neuronal differentiation potency of the pluripotent stem cells is enhanced. Increased neuronal differentiation potency can be confirmed by using as an index an increase in expression of nervous system markers (Sox2, nestin, Sox1, and the like) as compared with that before the start of step (1). Moreover, the expression of an undifferentiation marker may be utilized for evaluation of increased neuronal differentiation potency.

Usually, the cells after step (1) are once collected and then the process proceeds to the next culture (step (2)). The collection operation can be performed in the same manner as the collection operation during subculture.

### Step (2)

In this step, the cells obtained in step (1) are cultured in suspension in the presence of a TGF-β family inhibitor, a GSK3β inhibitor, FGF8, and a hedgehog signal agonist and under normal oxygen partial pressure to form a neurosphere. That is, the cells after step (1) are cultured using a medium to which a TGF-β family inhibitor, a GSK3β inhibitor, FGF8, and a hedgehog signal agonist are added and under the condition of normal oxygen partial pressure. Step (2) aims to induce differentiation along the neuron lineage. The features not specifically mentioned (usable basic medium, usable TGF-β family inhibitor, GSK3β inhibitor, other components that can be added to the medium, and the like) are the same as those in step (1), and the explanations thereof are omitted.

For example, flasks, tissue culture flasks, dishes, Petri dishes, tissue culture dishes, multi-dishes, microplates, microwell plates, micropores, multi-plates, multi-well plates, chamber slides, laboratory dishes, tubes, trays, culture bags, roller bottles, and the like can be used for suspension culture. In order to enable culture under non-adherent conditions, it is preferable to use a culture vessel having a non-cell-adherent culture surface. Examples of the culture vessel involved include culture vessels whose surfaces (culture surfaces) have been treated to be non-cell-adherent, and culture vessels whose surfaces (culture surfaces) have not undergone a treatment for improving the cell adhesiveness (for example, coating treatment with an extracellular matrix). It is only necessary to maintain the non-adherent state of the cells to the culture vessel in suspension culture. Static culture may be employed, or swirl culture or shaking culture may be employed.

Among the components added to the medium, the TGF-β family inhibitor and the GSK3β inhibitor are as described above. Also in this step, it is preferable to use SB431542 as the TGF-β family inhibitor and CHIR99021 as the GSK3β inhibitor. The concentration of the TGF-β family inhibitor in the medium when SB431542 is used is, for example, 0.5 µM to 20 µM, preferably 1 µM to 10 µM. Similarly, the concentration of the GSK3β inhibitor in the medium when CHIR99021 is used is, for example, 0.5 µM to 20 µM, preferably 1 µM to 10 µM.

FGF8 is a member of the fibroblast growth factor family. FGF8 is involved in the control of vertebrate brain formation and is required for regionalization to the midbrain. As long as the object of the present invention can be achieved, FGF8s derived from various mammals can be used. However, it is preferable to use FGF8 derived from the same origin (animal species) as that of the pluripotent stem cells to be used. Therefore, human FGF8 is preferably used when human pluripotent stem cells are used. The "human FGF8" means FGF8 having an amino acid sequence of FGF8 naturally expressed in the human body, and may be a recombinant. As a typical amino acid sequence of human FGF8, the NCBI accession number: NP_006110.1 (fibroblast growth factor 8 isoform B precursor [Homo sapiens].) can be exemplified. The concentration of FGF8 (amount to be added to the medium) is not particularly limited as long as the purpose of inducing differentiation along the neuronal lineage is achieved, but is, for example, 1 ng/ml to 5 µg/ml, preferably 10 to 500 ng/ml, more preferably 50 to 400 ng/ml. The optimum concentration can be set through preliminary experiments.

The hedgehog signal agonist is not particularly limited as long as it promotes a sonic hedgehog (SHH) signal. For example, purmorphamine (9-cyclohexyl-N-[4-(4-morpholinyl)phenyl]-2-(1-naphthalenyloxy)-9H-purin-6-amine) useful for inducing ventralization is preferably used. The concentration of purmorphamine (amount added to the medium) is not particularly limited as long as the purpose of inducing differentiation along the neuron lineage is achieved, but is, for example, 1 ng/ml to 5 µg/ml, preferably 10 to 500 ng/ml, more preferably 50 to 400 ng/ml. The optimum concentration can be set through preliminary experiments. SAG (N-methyl-N'-(3-pyridinylbenzyl)-N'-(3-chlorobenzo[b]thiophene-2-carbonyl)-1,4-diaminocyclohexane)can also be used as the hedgehog signal agonist. The concentration of SAG used (amount added to the medium) is not particularly limited as long as the purpose of inducing differentiation along the neuron lineage is achieved, but is, for example, 10 nM to 100 µM, preferably 100 nM to 10 µM, more preferably 100 nM to 2 µM. The optimum concentration can be set through preliminary experiments.

It is not essential that the concentrations of the respective components (TGF-β family inhibitor, GSK3β inhibitor, FGF8 and hedgehog signal agonist) be constant throughout the entire culture period, and the concentration(s) of a specific component (which may be two or more components) or all the components may change during the culture. For example, the addition of FGF8 and hedgehog signal agonist is started on the second to sixth days of step (2). According to this condition, rapid stimulation to cells can be relieved. Preferably, the addition of FGF8 and hedgehog signal agonist is started on the third to fifth days of step (2).

In order to promote differentiation induction along the neuron lineage, a medium to which a leukemia inhibitory factor (LIF) is also added is preferably used. As long as the object of the present invention can be achieved, LIFs derived from various mammals can be used. However, it is preferable to use LIF derived from the same origin (animal species) as that of the pluripotent stem cells to be used. Therefore, human LIF is preferably employed when human pluripotent stem cells are used. The concentration of the LIF is not particularly limited, but is, for example, 0.25 ng/ml to 1 µg/ml, preferably 1 ng/ml to 50 ng/ml, more preferably 5 ng/ml to 20 ng/ml. The optimum concentration can be set through preliminary experiments.

In order to promote differentiation induction along the neuronal cell lineage, a medium to which a bFGF (basic fibroblast growth factor) is also added is preferably used. The bFGF is also called FGF2. As long as the object of the present invention can be achieved, bFGFs derived from various mammals can be used. However, it is preferable to use bFGF derived from the same origin (animal species) as that of the pluripotent stem cells to be used. Therefore, human bFGF is preferably employed when human pluripotent stem cells are used. The "human FGF2" means FGF2 having an amino acid sequence of FGF2 naturally expressed in the human body. As a representative amino acid sequence of human FGF2, the NCBI accession number: NP_001997.5 (fibroblast growth factor 2 [Homo sapiens]) can be exemplified. The concentration of the bFGF is not particularly limited, but is, for example, 0.25 ng/ml to 1 µg/ml, preferably 1 ng/ml to 50 ng/ml, more preferably 3 ng/ml to 30 ng/ml. The optimum concentration can be set through preliminary experiments.

In order to suppress cell death, a medium to which a ROCK inhibitor (Rho-associated coiled-coil forming kinase/Rho-binding kinase) (for example, Y-27632 or Fasudil (HA-1077)) is also added is preferably used. The concentration of Y-27632 used as the ROCK inhibitor is, for example, about 1 µM to about 50 µM. The optimum concentration can be set through preliminary experiments.

The ROCK inhibitor strongly inhibits cell death when cells are in a dispersed state. Therefore, instead of using the ROCK inhibitor over the entire culture period of step (2), the cells may be treated in a medium containing the ROCK inhibitor only when seeding cells (i.e. at the start of culture) or when collecting and dispersing cells, for example, for subculture.

Preferably, a medium having a ciliary neurotrophic factor (CNTF), a brain-derived neurotrophic factor (BDNF), a neurotrophin 3 (NT-3), a fetal bovine serum, an N2 supplement, a B27 supplement, or the like added is used so that the use thereof is advantageous in differentiation induction along the neuron lineage. Incidentally, the N2 supplement is available from Gibco (product name N2 supplement (x100)) or the like, and the B27 supplement is available from Gibco (product name B27 supplement (x100))or the like.

Furthermore, any other component may be added to the medium as needed. Examples of the component which can be added include insulin, an iron source (e.g., transferrin), a mineral (e.g., sodium selenate), a saccharide (e.g., glucose), an organic acid (e.g., pyruvic acid or lactic acid), a serum protein (e.g., albumin), an amino acid (e.g., L-glutamine), a reducing agent (e.g., 2-mercaptoethanol), a vitamin (e.g., ascorbic acid or d-biotin), an antibiotic (e.g., streptomycin, penicillin, or gentamicin), and a buffer (e.g., HEPES).

In this step (2), suspension culture is performed to form a neurosphere. For example, Serum-free Floating culture of Embryoid Body-like aggregates with quick reaggregation (SFEB method/SFEBq method; Watanabe et al., Nature Neuroscience 8,288-296 (2005), WO 2005/123902), neurosphere method (Reynolds BA and Weiss S., Science, USA, 1992 Mar 27; 255 (5052): 1707-10) and the like can be employed.

In the present invention, step (2) is performed under normal oxygen partial pressure. In cell culture, the condition of a lowered oxygen concentration (low oxygen partial pressure/low oxygen concentration) may sometimes be used in consideration of the environment in the living body. However, the condition "under normal oxygen partial pressure" in the present invention is in contrast to such a special condition. That is, the condition "under normal oxygen partial pressure" is a condition in which the oxygen concentration is not intentionally adjusted. The oxygen concentration in the case of the condition "under normal oxygen partial pressure" is typically about 18% to about 22%, though it may vary depending on other conditions (humidity, coexisting CO₂ concentration, and the like).

Performing step (2) under normal oxygen partial pressure eliminates the need to set a special oxygen condition (typically, low oxygen environment) throughout the entire culture period (steps (1) to (3)) (i.e., all of steps (1) to (3) can be performed under normal oxygen partial pressure), and can suppress induction of differentiation into unnecessary cells such as glial cells, and thus is a highly practical preparation method.

Other culture conditions (culture temperature, CO₂ concentration, and the like) can be set as appropriate. The culture temperature is, for example, about 30 to 40°C, preferably about 37°C. The CO₂ concentration is, for example, about 1 to 10%, preferably about 5%.

The period of step (2) (culture period) is, for example, 7 days to 21 days, preferably 10 days to 16 days. If the culture period is too short or too long, the differentiation efficiency may be lowered. Also, if the culture period is excessively long, one of the effects of the present invention, that is, efficient preparation of dopamine neurons, can be impaired.

The formed neurosphere may be collected to dissociate, and then the dissociated cells may be subjected to further suspension culture. That is, subculture may be performed. However, the number of subcultures is preferably small, and the number of subcultures is set to 1 or 0 (that is, no subculture is performed). Such a small number of subcultures is advantageous in the preparation of dopamine neurons in a short period of time, and is considered to be also effective in avoiding promotion of unintended differentiation induction (for example, induction of differentiation into glial cells). On the other hand, since subculture is effective in improving the cell purity, it can be said that the subculture is optimally performed once. When subculture is performed once, the subculture is preferably performed on the sixth to tenth days from the start of step (2). In addition, when collecting the neurosphere during subculture, it is preferable to prevent contamination of the cells adhering to the surface of the culture vessel. Such an operation can contribute to the improvement of the preparation efficiency and purity of dopamine neurons.

### Step (3)

The neurosphere formed by step (2) contains undifferentiated cells of the nervous system and undifferentiated cells of the midbrain system. In step (3), the neurosphere are collected to induce differentiation thereof into dopamine neurons. Media and culture conditions suitable for inducing differentiation into dopamine neurons are known. Regarding basic culture methods and operations, a protocol provided by ThermoFisher (published on the ThermoFisher website) or the like can be referred to. Specifically, for example, differentiation into dopamine neurons is induced by adherent culture in a medium containing a γ-secretase inhibitor, a neurotrophic factor, ascorbic acid, TGF-β3 and cAMP or a cAMP analog. Preferably, the γ-secretase inhibitor used is N-[N-(3,5-difluorophenacetyl-L-alanyl)]-S-phenylglycine t-butyl ester, the neurotrophic factor used is a brain-derived neurotrophic factor (BDNF) and a glial cell-derived neurotrophic factor (GDNF), and the cAMP analog used is diptyryl cAMP.

Typically, the neurosphere formed in step (2) is collected to dissociate (to form single cells), and the dissociated cells are seeded in a culture vessel and cultured. Instead of such dispersion culture, the collected neurosphere may be subjected to adherent culture as a cell aggregate. In this case, normally, if culture is continued, some of cells migrate from the neurosphere to the surrounding, and dopamine neurons can be observed in the migrated cells.

For example, dishes, Petri dishes, tissue culture dishes, multi-dishes, microplates, microwell plates, multi-plates, multi-well plates, chamber slides, laboratory dishes, and the like can be used in this adherent culture. In order to enhance the adhesiveness of the cells to the culture surface, it is preferable to use a culture vessel coated with Matrigel™ (BD), poly-D-lysine, poly-L-lysine, collagen, gelatin, laminin, heparan sulfate proteoglycan, entactin, or a combination of two or more thereof.

Other culture conditions such as culture temperature, CO₂ concentration, and O₂ concentration can be set as appropriate. The culture temperature is, for example, about 30 to 40°C, preferably about 37°C. The CO₂ concentration is, for example, about 1 to 10%, preferably about 5%. Moreover, culture may be performed under normal oxygen partial pressure.

The period (culture period) of step (3) is not particularly limited. For example, the culture is performed for 5 days or longer, preferably 7 days or longer. Although culture for an excessively long period can cause exhaustion of cells, decrease in activity, cell death, or the like, differentiation/maturation generally progresses as the culture period is longer. Therefore, the culture period is, for example, set to 5 days to 21 days, although the upper limit of the culture period in this step is not particularly limited. The cells may be subjected to subculture as necessary. For example, the cells are collected at a stage where they are brought in a subconfluent or confluent state, a part of the cells is seeded in another culture vessel, and culture is continued.

By step (3), dopamine neurons are obtained. Dopamine neurons can be identified or confirmed using, as an index, expression of dopamine markers (tyrosine hydroxylase, dopamine transporter), FOXA2 as a midbrain marker, or the like, or by evaluation of the dopamine production ability (see the Examples below).

According to the preparation method of the present invention, dopamine neurons can be obtained from pluripotent stem cells in about 21 days to 30 days, though it may vary depending on the type and state of the cells used and the culture conditions in each step.

### 2. Use of dopamine neurons

A second aspect of the present invention relates to use of dopamine neurons obtained by the preparation method of the present invention. The dopamine neurons of the present invention can themselves be used as therapeutic agents or transplant materials for various neurological diseases. The envisioned applications of the dopamine neurons of the present invention are typically central nervous system diseases to be treated with dopamine drugs, such as schizophrenia, bipolar disorder, attention deficit hyperactivity, autism spectrum disorder, and Parkinson's disease. Prior to the application to transplantation medicine or the like, the prepared cells may be refined or purified using cell surface markers, morphology, secretory substances, and the like as indices.

On the other hand, the dopamine neurons of the present invention are also useful as experimental tools, and can be applied, for example, to various assays (drug screening systems, drug efficacy evaluation systems, drug response assays, and the like) in the development of drugs (therapeutic agents, preventive agents) for various neurological diseases, various assays (gene expression analysis, proteomics analysis, morphological analysis, neuroelectrophysiological analysis, and the like) in researches aimed at elucidating/understanding the mechanisms for onset and progression of various neurological diseases, and evaluation of neurotoxicity (toxicity evaluation system). It is also applicable to an *in vivo* assay using a non-human animal. The usefulness of the dopamine neurons obtained by the preparation method of the present invention for various assays is demonstrated in the Examples which will be described later.

### [Examples]

### <Establishment of novel method for preparing dopamine neurons and research using the same>

As a pathological hypothesis of schizophrenia (SCZ) and autism spectrum disorder (ASD), it is thought that neurodevelopmental disorder plays an important role. Not only in ASD whose characteristics have already become apparent from the developmental stage, but also in SCZ, it has been reported that there are some cognitive dysfunctions and neurophysiological or neuroimaging changes before the onset without obvious psychiatric symptoms, and that, as a result of studies using the postmortem brain of SCZ and ASD, there is a failure in brain construction caused by neurons. These reports suggest that neurodevelopmental disorders that begin in the fetal period are causes of the onset of SCZ and ASD, but details of the pathologic conditions in the brain of patients with SCZ and ASD are unknown.

Genomic studies of SCZ and ASD have identified a plurality of variants in genes involved in neurodevelopment in both diseases, one of which is a variant in RELN. The protein reelin encoded by RELN is a large secreted protein and is essential for the formation of the layer structure of the developing brain. In humans, a RELN homozygous deletion mutation causes spondylosis with developmental delay, and a decrease in reelin has been reported to be associated with the onset of neurodevelopmental disorders. Similarly, reeler mice, which are Rein-mutant mice, exhibit disordered brain layer structure and abnormal behavior, and abnormal migration directions of individual neurons have also been reported. These reports suggest that even in humans, a decrease in reelin in the brain may cause unstable neuronal migration, which is expected to cause neurodevelopmental disorders related to SCZ and ASD.

There are several species of neurons that express Reelin. Among them, tyrosine hydroxylase (TH)-positive dopamine neurons have been reported, in researches using mice, to express reelin only in a limited period before and after birth. Although reelin is expressed only in such a limited period, abnormalities in dopamine neurons have been confirmed in Rein-mutated reeler mice. On the other hand, the presence of many reports that the dopamine system is involved in the pathologic conditions of SCZ and ASD suggests the possibility that, through investigation of the relationship between dopamine neurons and reelin, clues to elucidate the mechanism for onset of SCZ and ASD may be obtained.

Therefore, attempts were made in this study to establish iPS cells from a total of 2 people, i.e., a SCZ patient and his/her healthy family member with RELN deletion and to artificially prepare RELN-deleted iPS cells (RELN-deleted isogenic lines) through genome editing. At that time, in view of future development and applications, the method for preparing dopamine neurons from iPS cells (inducing differentiation) was improved.

As will be indicated below, all the iPS cells could be differentiated into TH-positive dopamine neurons by the successfully-established novel preparation method. Live imaging analysis revealed that dopamine neurons derived from the RELN-deleted iPS cells exhibit an abnormal migration direction instead of an abnormal migration distance. These findings contribute to the elucidation of the effects of reelin dysfunction in the developing human brain, and RELN variant is thought to reflect the molecular pathologic conditions leading to SCZ.

### 1. Material and method

### (1) Persons from which iPS cells were established

Two (2) persons with RELN deletion (parent and child; the child was a schizophrenic patient, and his/her mother was a healthy person)

Two (2) healthy controls (2 healthy males with no RELN deletion)

### (2) Establishment of iPS cells

A healthy female control (201B7) was obtained from RIKEN BioResource Center (BRC). The absence of genomic abnormalities including RELN deletion was confirmed in advance. Other iPS cells were established, using episomal vectors, from peripheral lymphocytes in accordance with the previous report (Okita, K. et al. A more efficient method to generate integration-free human iPS cells. Nature methods 8, 409-412 (2011)). The established iPS cells were cultured on feeder cells (mitomycin-C-treated mouse embryonic fibroblasts: MEFs) using an iPS cell medium (DMEM/F12 containing 20% KSR, 2 mM L-glutamine, 0.1 mM non-essential amino acids, 2-mercaptoethanol, penicillin/streptomycin, and bFGF). In feeder-free culture, the iPS cells were cultured on a culture dish coated with Matrigel™ (BD). As the medium, an iPS cell medium (MEF-conditioned medium) exposed to feeder cells overnight was used.

### (3) Array CGH

Genomic DNA was isolated from peripheral blood or iPS cells cultured under feeder-free conditions. Array CGH was performed in accordance with the previous report (Kushima, I. et al. High-resolution copy number variation analysis of schizophrenia in Japan. Molecular psychiatry (2016)).

### (4) Embryoid body (EB) formation and confirmation of differentiation into three germ layers in vitro

The iPS cells were dispersed with TrypLE™ select (Thermo Fisher Scientific Inc.), and then cultured in suspension in a DMEM/F12 medium (containing 5% KSR, 2 mM L-glutamine, 0.1 mM non-essential amino acids, 2-mercaptoethanol, Y27632, and penicillin/streptomycin) for 7 days to form EBs. Thereafter, the EBs were seeded on a gelatin-coated culture dish in a 10% FBS DMEM medium, and differentiation was induced spontaneously over 7 days.

### (5) CRISPR/Cas9 system

A Cas9 expression vector and an sgRNA expression vector were obtained from Addgene.

### (6) Introduction of CRISPR into HEK293FT and human iPS cells

HEK293FT was cultured in a 10% DMEM medium. The Cas9 expression vector and sgRNA expression vector were co-transfected using Lipofectamine 3000, and selection with puromycin was performed after 48 hours. In the case of human iPS cells, 201B7 and CON779 healthy control lines cultured under feeder-free conditions were used. After pretreatment with 10 µM Y-27632, the iPS cells were dispersed using TrypLE™ select (Thermo Fisher Scientific Inc.). Thereafter, the Cas9 expression vector and sgRNA expression vector were co-transfected using FuGENE (registered trademark) HD (Promega), and the cells were seeded on a 6-well plate coated with Matrigel™ (BD) at 1 × 10⁶ cells/well. Selection with puromycin was performed after 24 hours.

### (7) T7EI assay

In order to examine the cleavage activity of sgRNA, a T7EI assay was performed. The target region was amplified by PCR, heat denatured (for 2 min at 95°C) and re-annealed (temperature decreased from 85°C to 25°C at - 0.1°C/sec). Thereafter, DNA was cleaved with T7EI, and the product was electrophoresed on a 1.5% gel.

### (8) Neuronal differentiation

Differentiation into dopamine neurons was performed by a method established by improving the previously reported method (Fujimori, K. et al. Modeling neurological diseases with induced pluripotent cells reprogrammed from immortalized lymphoblastoid cell lines. Molecular brain 9, 88 (2016)). First, iPS cells were cultured in an iPS cell medium to which SB431542 (3 µM), CHIR99021 (3 µM), and dorsomorphin (3 µM) were added for 7 days (from Day 0 to Day 7). Then, the iPS cells which were dispersed by TrypLE™ select (Thermo Fisher Scientific Inc.) and caused to pass through a cell strainer were cultured in suspension in a neurosphere medium (a medium (MHM medium) obtained by adding, to a DMEM/F12 medium, 1 × N2 supplement, 0.6% glucose, penicillin/streptomycin and 5 mM HEPES, to which 1 × B27 supplement, 20 ng/ml bFGF, 10 ng/ml human LIF, 10µM Y27632, 3µM CHIR99021, 2µM SB431542, 100 ng/ml FGF8, and 1µM purmorphamine were added) for 2 weeks. As a result, neurospheres were formed (Day 7 to Day 21). FGF8 and purmorphamine were added from Day 10. On Day 14, the neurospheres were collected and dispersed to form single cells, and then the cells were cultured in suspension again to form neurospheres again (secondary neurospheres).

On Day 21, the neurospheres were seeded on a Matrigel™ (BD)-coated or poly-L-ornithine/laminin-coated culture dish, and cultured in a medium for dopamine neurons (MHM medium to which B27 supplement, 10 µM DAPT, 20 ng/ml BDNF, 20 ng/ml GDNF, 0.2 mM ascorbic acid, 1 ng/ml TGF-β3, and 0.5 mM dbcAMP were added) to induce differentiation into dopamine neurons (on Day 21 or later). All cultures were performed in a normal CO₂ incubator (5% CO₂; the oxygen concentration was 18.5% to 19.5% (not adjusted)).

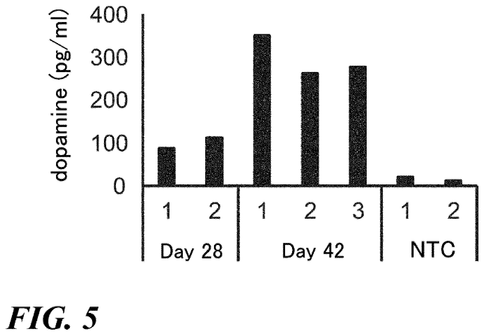

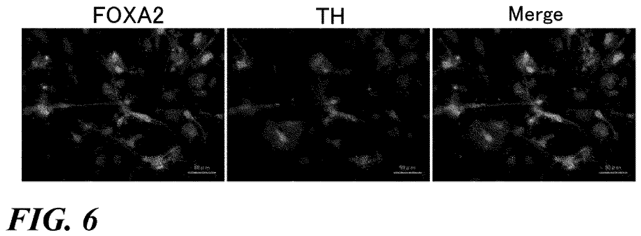

According to the above protocol, healthy iPS cells were induced into dopamine neurons to measure the dopamine concentrations in the culture supernatants on Day 28 and Day 42 (Fig. 5). In addition, the cells on Day 28 were fixed and immunostained with a midbrain marker (FOXA2) and a dopamine neuron marker (TH) (Fig. 6). An increase in dopamine amount over time was observed (Fig. 5), confirming that the induction of differentiation into dopamine neurons could be achieved, and that maturation progressed as the culture period was increased. In addition, since double-positive cells are observed (Fig. 6), it can be seen that they have been induced into midbrain dopamine neurons.

### (9) Neuronal migration test

The secondary neurospheres (Day 21) were seeded one by one on Matrigel^{M} (BD)-coated culture dishes and cultured in a medium for dopamine neurons. A video was shot with IncuCyte (registered trademark) (ESSEN BIOSCIENCE). For cell tracking, images were shot continuously every 15 minutes for a total of 4 hours, 48 hours to 52 hours after seeding, and analyzed using ImageJ. The migration distance was calculated based on the XY coordinates at each shooting point.

MATLAB (Mathworks, Natick) was used to analyze the migration angle of each cell. The position of the cell at each time point was obtained from the XY coordinates to measure the movement angle from the reference point. The cell position on the nth image was defined as Cellⁿ, and the start point (= cell position 48 hours after seeding) was defined as Cell⁰. The average value of the cell migration direction for 4 hours was defined as the standard axis. The migration angle of each cell at each time point was an angle from the standard axis. When the cells did not move between the two consecutive images, they were excluded from the target for analysis.

### (10) Immunocytochemistry

Cells were fixed with 4% paraformaldehyde (15 minutes, room temperature). The cells were soaked in PBS containing 1% BSA and 0.3% TritonX-100 for 1 hour for membrane permeation and blocking treatment. Thereafter, a primary antibody reaction (4°C, overnight) was performed. After washing with PBS, the cells were caused to react with a fluorescently-labeled secondary antibody (room temperature, 1 hour). TRA-1-60 (abcam), NANOG (abcam), SOX17 (R & D systems), αSMA (R & D systems), TUJ1 (SIGMA), TH (Chemicon) and Reelin (MBL) were used as the primary antibodies. BZ-9000 (KEYENCE) or LS780 (Zeiss) was used for capturing images.

### (11) Measurement of dopamine concentration in medium

In order to examine the dopamine production ability of the dopamine neurons obtained by differentiation induction, the dopamine concentration in the culture supernatant was measured using an ELISA kit (DLD EA608-96). The culture supernatant on Day 28 was used as a sample. The culture supernatants obtained from at least 3 independent experiments (cultures) were used.

### (12) DNA microarray and quantitative PCR

Total RNAs were extracted using RNeasy Plu Mini Kit. DNA microarray was performed using SurePrint G3 Hmm GE 8x60K V2 Microarray Kit (Agilent Technology), and analysis was performed by GeneSpring GX software program (version 13; Aglilent Technology). For a reverse transcription reaction, High-Capacity cDNA Transcription Kit (Applied Biosystems) was used. Gene expression analysis by quantitative PCR was performed at 7900HT (Applied Biosystems) using KAPA SYBR Fast qPCR Kit (KAPA BIOSYSTEMS).

### (13) Statistical analysis

For comparison of the average values, the Student t-test (two-sided test) was used for comparison between two groups, and the Dunnett's method was used after the ANOVA test for comparison among three groups. Comparison of the distributions was made using the Pearson's Chi-square test. In all cases, p <0.05 was considered significant.

### 2. Results

### (1) Preparation of RELN-deleted iPS cells

Using peripheral lymphocytes of 2 healthy controls (CON779 and CON1004) and 2 RELN-deleted persons (SCZ339 and FAM258), iPS cells were established by the episomal vector method. For the respective iPS cells obtained, the expression of pluripotency markers (NANOG and TRA-1-60) and the tridermic differentiation potency (endoderm: SOX17, mesoderm: αSMA, ectoderm: Tuj1) were confirmed (the results of FAM258 are shown in Figs. 1A and B).

In order to evaluate the genomic consistency of the established iPS cells, genome analysis was performed through array CGH and TaqMan Copy Number assay. RELN deletion was confirmed in the RELN-deleted iPS cells (Fig. 1C). Chromosome aneuploidy (= CNV) in iPS cells is a phenomenon that can occur during the establishment process. Because of concerns about the impact on future analyses, iPS cell lines in which CNV other than RELN deletion was found were excluded from the target for analysis. However, for CON779, 20q11.21 duplication was newly found, but it was used for analysis (because this CNV is frequently identified also in human ES cells).

In this study, no one with RELN deletion could be identified other than NS339 and NF258. Since these two persons are parent and child, it is considered difficult to determine whether the phenotype observed in the iPS cells derived from these two persons is due to RELN deletion or is specific to them. Thus, RELN-deleted isogenic iPS cell lines were artificially prepared using the CRISPR/Cas9 system. Four types of single-stranded guide RNAs (sgRNAs) were designed in the RELN deletion region (Fig. ID). As a result of the T7EI assay, it was found that sgRNA #4 had the highest cleavage efficiency (Fig. IE), and thus it was decided to prepare an isogenic line using sgRNA #4. For reproducibility of the results and in order to eliminate the possibility of off-targeting, isogenic lines were prepared from two healthy controls (201B7 and CON779), respectively. As shown in Fig. IF, a plurality of isogenic lines could be prepared. All the lines remained to hold the three germ layers differentiation potency (Fig. 1G). Furthermore, the possibility of off-target was investigated using CCTop. As a result, the cleavage region expected in the exon region was only the target RELN (data not shown).

### (2) Human iPS cell-derived dopamine neurons express reelin

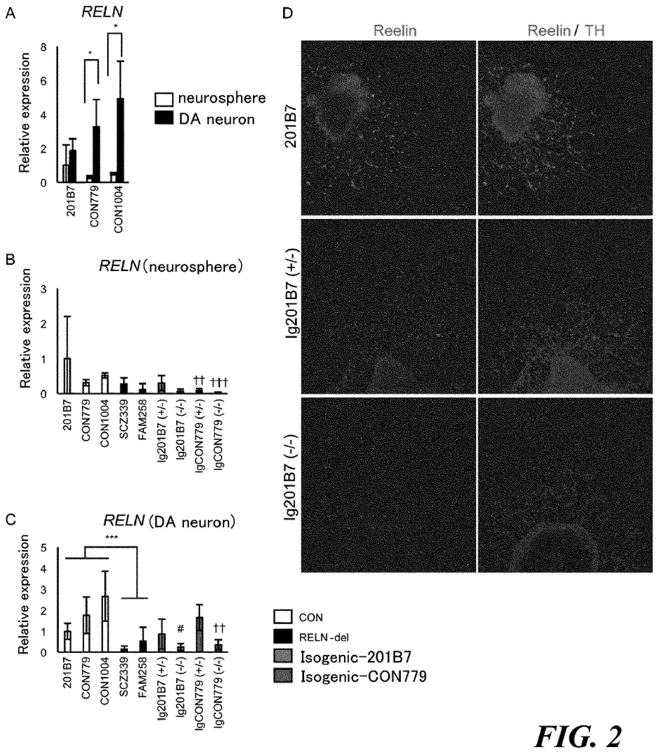

In order to examine whether the iPS cell-derived neural cells expressed reelin, the expression levels of RELN mRNA in neurospheres (Day 21) and dopamine neurons (Day 28) were first analyzed by quantitative RT-PCR. In the healthy control group, the RELN expression in the dopamine neurons was higher than that in the neurospheres (Fig. 2A). This suggests the possibility that the dopamine neurons express reelin. On the other hand, in the nervous system, particularly the dopamine neurons, derived from the RELN-deleted iPS cells, the RELN mRNA expression was observed to be lowered as compared with that in the healthy control group.

Next, the expression of reelin in the dopamine neurons was examined using immunocytochemistry. The parent line 201B7 and isogenic lines (heterozygous deletion and homozygous deletion) were used. In 201B7, the expression of reelin was confirmed in the TH-positive neurons. In the heterozygous deletion line 201B7 (+/-), the expression of reelin was confirmed although the signal was somewhat weak. However, no reelin signal was detected in the homozygous deletion line 201B7 (-/-) (Fig. 2D). From the above results, it was clarified that reelin is expressed in the TH-positive dopamine neurons also in humans, and that abnormal expression of reelin is observed in RELN-deleted lines, as previously reported on mice.

### (3) Similarly to healthy control iPS cells, RELN-deleted iPS cells differentiate into TH-positive neurons

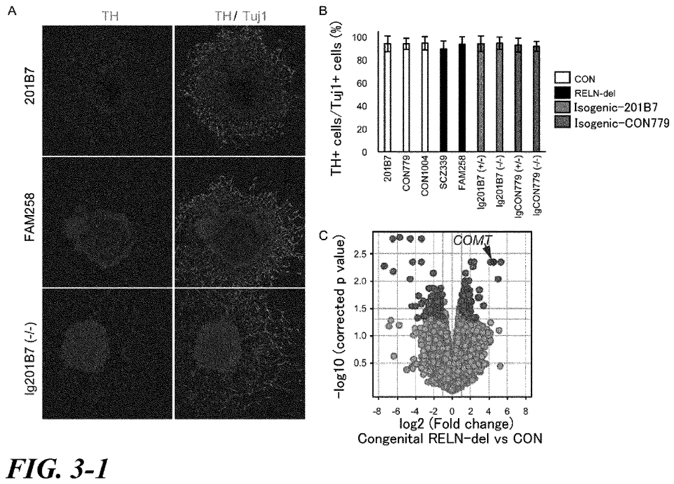

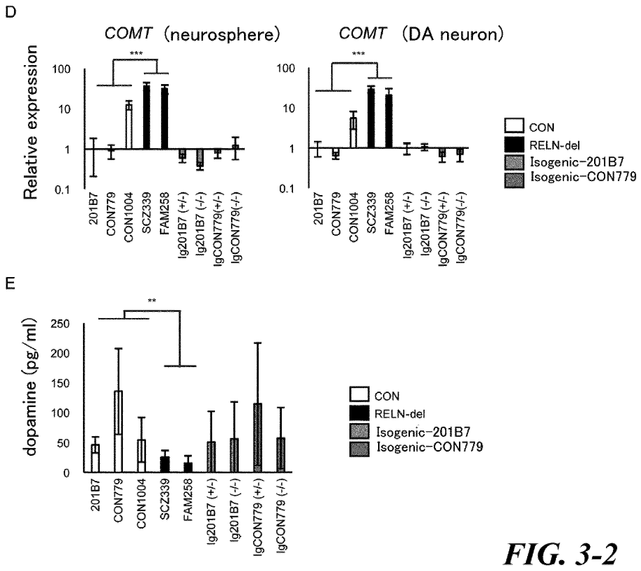

According to the previous reports (Hook, V. et al. Human iPSC neurons display activity-dependent neurotransmitter secretion: aberrant catecholamine levels in schizophrenia neurons. Stem cell reports 3, 531-538 (2014), Robicsek, O. et al. Abnormal neuronal differentiation and mitochondrial dysfunction in hair follicle-derived induced pluripotent stem cells of schizophrenia patients. Molecular psychiatry 18, 1067-1076 (2013)), iPS cells derived from schizophrenic patients show abnormal differentiation into dopamine neurons. Therefore, the healthy control iPS cells and the RELN-deleted iPS cells were compared in terms of the differentiation potency into TH-positive cells. As shown in Figs. 3A and B, all the iPS cells differentiated into TH-positive cells with high efficiency, regardless of RELN deletion, according to the newly-established protocol. Fig. 3B shows the results 48 hours after the start of step (3). Culture was continued, and the TH-positive rates, when measured for 201B7, CON779, and CON1004 on Day 7 from the start of step (3), were all over 85%.

Next, comprehensive gene expression comparative analysis of the healthy control iPS cells (CONT: 201B7 and CON779) and the congenital RELN-deleted parent and child (RELN-del: SCZ339 and FAM258) was performed by DNA microarray using neurospheres. Among the genes involved in dopamine metabolism, the expression of COMT was remarkably high in the RELN-del group (more than 20 times) (Fig. 3C). COMT is a gene encoding an enzyme called catechol-O-methyltransferase, and this enzyme is also involved in the adjustment of the dopamine concentration in the brain. In order to clarify whether high expression of COMT observed in the RELN-deleted parent and child was due to RELN deletion, the RELN-deleted isogenic lines were also used and analyzed by quantitative PCR. As a result, high expression of COMT was observed only in the RELN-deleted parent and child (SCZ339 and FAM258), but not in the RELN-deleted isogenic lines.

In order to examine the dopamine production ability, the dopamine concentration in the culture supernatant was measured using an ELISA kit. As a result, the congenital RELN-deleted parent and child showed a significant decrease in dopamine amount as compared with the healthy control group. On the other hand, both of the isogenic lines showed a dopamine amount equivalent to that of the parent line (Fig. 3E).

### (4) RELN-deleted iPS cell-derived neurons show abnormal migration direction

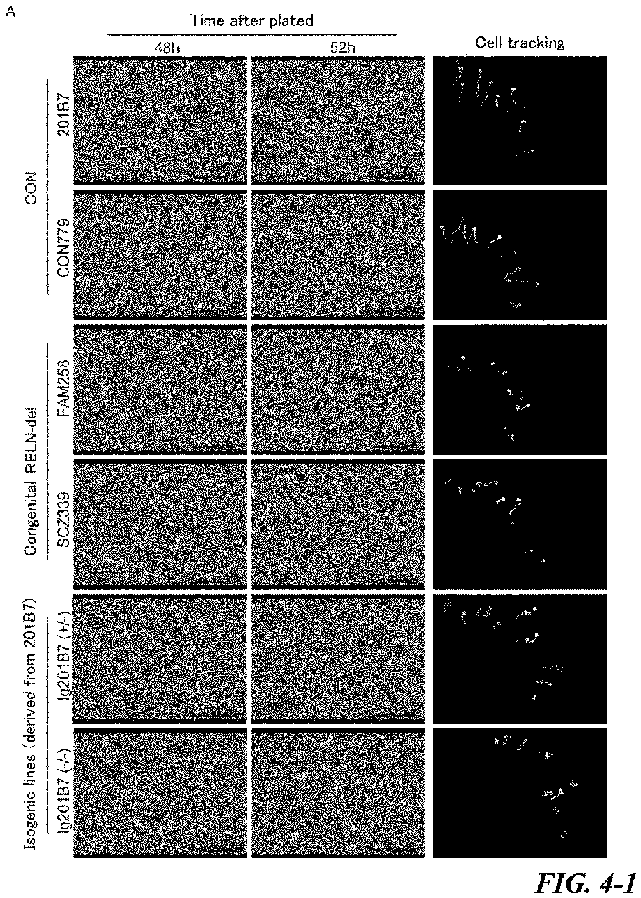

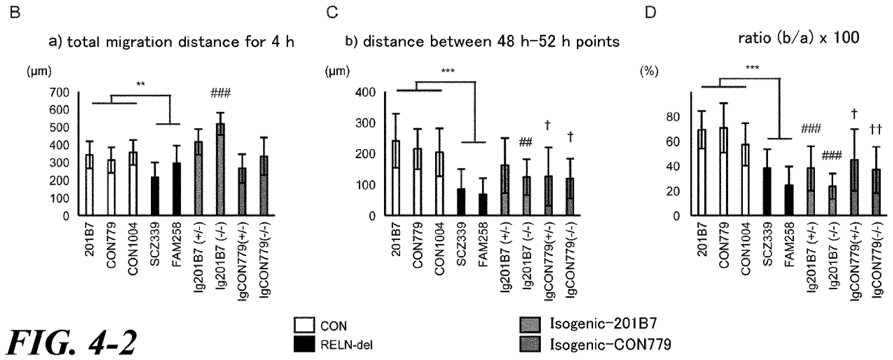

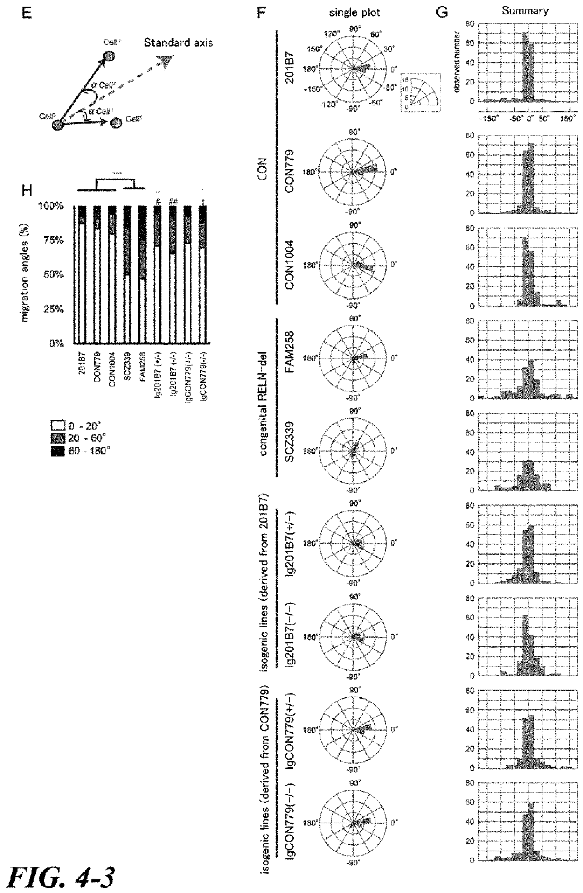

In the developing brain of reeler mice, dopamine neurons have been reported to exhibit an abnormal migration direction (Bodea, G.O. et al. Reelin and CXCL12 regulate distinct migratory behaviors during the development of the dopaminergic system., England) 141, 661-673 (2014)). A cell tracking assay using RELN-deleted neurons was performed to examine whether a similar abnormality occurred in humans. Healthy control (201B7, CON779, CON1004)-derived dopamine neurons migrate straight from the marge of the neurosphere to the outside, whereas RELN-deleted neurons showed an abnormality in the migration directional properties (Fig. 4A). For objective evaluation, quantitative analysis was performed for each group. When compared with the total movement distance of 4 hours, the RELN-deleted parent and child showed a significantly lower value than that of the healthy control group (Fig. 4B). On the other hand, no difference was observed in the isogenic lines except the homozygous deletion Ig201B7 (-/-). When the distance between two points, i.e., the start point (48h) and the end point (52h) of 4 hours was quantified, the RELN-deleted parent-child group showed the shortest distance, and the isogenic line also showed a distance shorter than that of the parent line (Fig. 4C). From this, it is expected that the RELN-deleted line does not migrate straight. In fact, when taking the ratio of the total movement distance and the distance between the two points (distance between the two points/total movement distance), a significant low value was observed in all the groups with RELN deletion, and it became clear that the RELN-deleted neurons lost the migration directional properties.

In order to analyze the neuronal migration direction in more detail, the migration angle at each point of each cell was measured. The measurement metrics are shown in Fig. 4E. The migration angle at Cellⁿ (αCellⁿ) was defined as the angle formed by the standard axis and the position vector at Cellⁿ. A positive angle indicates counterclockwise and a negative angle indicates clockwise. As shown in Figs. 4F and G, the angle at each point in the healthy control group was almost -20° <αCellⁿ <20 ° (201B7: 87%, CON779: 83%, CON1004: 80%). On the other hand, in the RELN-deleted group, SCZ339: 50%, FAM258: 47%, Ig201B7 ((+/-): 71%, Ig201B7 (-/-): 65%, IgCON779 (+/-): 73%, and IgCON779 (-/-): 70%. Due to RELN deletion, the normal directivity of neuronal migration direction was lost. Also in comparison of the distributions based on the angle, a significant change in angle due to RELN deletion was also observed (Fig. 4G). From the above, it was clarified that a decrease in reelin causes random migration of human neurons.

### 3. Conclusion

Variants that occur in the reelin gene (RELN) involved in neurodevelopment are thought to be involved in the onset of SCZ and ASD, but details of the molecular mechanisms leading to the onset have not been clarified. To address this issue, iPS cells were established from people having a congenital RELN deletion, and RELN-deleted isogenic lines were prepared using genome editing. Differentiation of the iPS cells into TH-positive neurons was induced using the newly-established protocol to examine their characteristics. First, when the differentiation of the established iPS cells into TH-positive neurons were induced, the RELN-deleted lines showed reduced expression of reelin as compared with the control lines. However, the efficiency of differentiation into TH-positive neurons was not affected by the presence or absence of RELN deletion, and induction was achieved with high efficiency in all the groups. In other words, using the established protocol, it was possible to make analysis using highly homogenous TH-positive cells, regardless of the presence or absence of RELN deletion. When the migration of the RELN-deleted TH-positive neurons was analyzed, the greatest feature compared to the control neurons was that the neuronal migration direction could not be controlled. As a result of real-time live imaging observation, it was confirmed that the control neurons showed a constant migration direction, whereas the RELN-deleted neurons showed an unstable migration direction. This was also reconfirmed by polar coordinate histogram analysis for quantifying the angle of the migration direction. The results are thought to have elucidated a part of the function of reelin in the human brain, and are expected to mimic the vulnerability phenomenon of the onset of mental disorders. RELN-deleted iPS cells will bring new insights into elucidation tools for human brain developmental disorders and may be useful for therapeutic agent development.

### [Industrial Applicability]

According to the preparation method of the present invention, it is possible to prepare dopamine neurons from pluripotent stem cells specifically/efficiently and, besides, in a short period of time even without using a special device. The dopamine neurons obtained by the preparation method of the present invention can be used as therapeutic agents or transplant materials for various neurological diseases. They are also useful as experimental tools and can be used in various assays.

The present invention is not limited to the description of the embodiments and examples of the present invention at all. Various modifications that can be easily achieved by those skilled in the art without departing from the claims also fall within the scope of the present invention. The contents of the articles, patent laid-open publications, patent publications, and the like specified herein shall be cited by incorporation in their entity.
## Claims

1. A method for preparing dopamine neurons, comprising the following steps (1) to (3):
(1) culturing pluripotent stem cells in the presence of a TGF-β family inhibitor, a GSK3β inhibitor, and a BMP inhibitor;
(2) suspension-culturing the cells obtained in step (1) in the presence of a TGF-β family inhibitor, a GSK3β inhibitor, FGF8, and a hedgehog signal agonist and under normal oxygen partial pressure to form a neurosphere; and
(3) collecting the neurosphere to induce differentiation of the cells into dopamine neurons.

2. The preparation method according to claim 1, wherein the pluripotent stem cells are induced pluripotent stem cells.

3. The preparation method according to claim 1 or 2, wherein the pluripotent stem cells are human cells.

4. The preparation method according to any one of claims 1 to 3, wherein the TGF-β family inhibitor is 4-[4-(1,3-benzodioxol-5-yl)-5-(2-pyridinyl)-1H-imidazol-2-yl]-benzamide or a hydrate thereof.

5. The preparation method according to any one of claims 1 to 4, wherein the GSK3β inhibitor is 6-[[2-[[4-(2,4-dichlorophenyl)-5-(4-methyl-1H-imidazol-2-yl)-2-pyrimidinyl]amino] ethyl] amino] nicotinonitrile.

6. The preparation method according to any one of claims 1 to 5, wherein the BMP inhibitor is 6-[4-(2-piperidin-1-ylethoxy)phenyl]-3-pyridin-4-ylpyrazolo[1,5-a]pyrimidine.

7. The preparation method according to any one of claims 1 to 6, wherein the hedgehog signal agonist is 9-cyclohexyl-N-[4-(4-morpholinyl)phenyl]-2-(1-naphthalenyloxy)-9H-purin-6-amine.

8. The preparation method according to any one of claims 1 to 7, wherein induction of differentiation into unnecessary cells such as glial cells is suppressed by culturing in suspension under the normal oxygen partial pressure in step (2).

9. The preparation method according to any one of claims 1 to 8, wherein the number of passages in step (2) is 0 or 1.

10. The preparation method according to claim 9, wherein promotion of unintended differentiation induction is avoided due to a small number of passages.

11. The preparation method according to any one of claims 1 to 10, wherein the culture period in step (1) is 4 days or longer.

12. The preparation method according to any one of claims 1 to 11, wherein all of steps (1) to (3) are carried out under the normal oxygen partial pressure.

13. The preparation method according to any one of claims 1 to 12, wherein the normal oxygen partial pressure is a condition where the oxygen concentration is 18% to 22%.

14. The preparation method according to any one of claims 1 to 13, wherein the culture in step (2) is performed under conditions where LIF, bFGF, and a ROCK inhibitor are further present.

15. The preparation method according to any one of claims 1 to 14, wherein the culture period in step (2) is 7 days to 21 days.

16. The preparation method according to any one of claims 1 to 15, wherein step (3) comprises adherent culture in the presence of a γ-secretase inhibitor, a neurotrophic factor, ascorbic acid, TGF-β3 and cAMP or a cAMP analog.

17. The preparation method according to claim 16, wherein the γ-secretase inhibitor is N-[N-(3,5-difluorophenacetyl-L-alanyl)]-S-phenylglycine t-butyl ester, wherein the neurotrophic factor is a brain-derived neurotrophic factor (BDNF) and a glial cell-derived neurotrophic factor (GDNF), and wherein the cAMP analog is diptyryl cAMP.

18. The preparation method according to claim 16 or 17, wherein the culture period in step (3) is 5 days to 21 days.

19. A dopamine neuron obtained by the preparation method according to any one of claims 1 to 18.

20. An *in vitro* assay using the dopamine neuron according to claim 19.


[Sequence Listing]

SEQUENCE LISTING

<110>  NATIONAL UNIVERSITY CORPORATION NAGOYA UNIVERSITY

<120>  METHODS FOR PREPARING DOPAMINERGIC NEURONS

<130>  NU17001P

<150>  JP P2017-082600
<151>  2017-04-19

<160>  10

<170>  PatentIn version 3.5

<210>  1
<211>  215
<212>  PRT
<213>  Homo sapiens

<400>  1

Met Gly Ser Pro Arg Ser Ala Leu Ser Cys Leu Leu Leu His Leu Leu
1               5                   10                  15

Val Leu Cys Leu Gln Ala Gln Val Thr Val Gln Ser Ser Pro Asn Phe
            20                  25                  30

Thr Gln His Val Arg Glu Gln Ser Leu Val Thr Asp Gln Leu Ser Arg
        35                  40                  45

Arg Leu Ile Arg Thr Tyr Gln Leu Tyr Ser Arg Thr Ser Gly Lys His
    50                  55                  60

Val Gln Val Leu Ala Asn Lys Arg Ile Asn Ala Met Ala Glu Asp Gly
65                  70                  75                  80

Asp Pro Phe Ala Lys Leu Ile Val Glu Thr Asp Thr Phe Gly Ser Arg
                85                  90                  95

Val Arg Val Arg Gly Ala Glu Thr Gly Leu Tyr Ile Cys Met Asn Lys
            100                 105                 110

Lys Gly Lys Leu Ile Ala Lys Ser Asn Gly Lys Gly Lys Asp Cys Val
        115                 120                 125

Phe Thr Glu Ile Val Leu Glu Asn Asn Tyr Thr Ala Leu Gln Asn Ala
    130                 135                 140

Lys Tyr Glu Gly Trp Tyr Met Ala Phe Thr Arg Lys Gly Arg Pro Arg
145                 150                 155                 160

Lys Gly Ser Lys Thr Arg Gln His Gln Arg Glu Val His Phe Met Lys

165                 170                 175

Arg Leu Pro Arg Gly His His Thr Thr Glu Gln Ser Leu Arg Phe Glu
            180                 185                 190

Phe Leu Asn Tyr Pro Pro Phe Thr Arg Ser Leu Arg Gly Ser Gln Arg
        195                 200                 205

Thr Trp Ala Pro Glu Pro Arg
    210                 215

<210>  2
<211>  288
<212>  PRT
<213>  Homo sapiens

<400>  2

Met Val Gly Val Gly Gly Gly Asp Val Glu Asp Val Thr Pro Arg Pro
1               5                   10                  15

Gly Gly Cys Gln Ile Ser Gly Arg Gly Ala Arg Gly Cys Asn Gly Ile
            20                  25                  30

Pro Gly Ala Ala Ala Trp Glu Ala Ala Leu Pro Arg Arg Arg Pro Arg
        35                  40                  45

Arg His Pro Ser Val Asn Pro Arg Ser Arg Ala Ala Gly Ser Pro Arg
    50                  55                  60

Thr Arg Gly Arg Arg Thr Glu Glu Arg Pro Ser Gly Ser Arg Leu Gly
65                  70                  75                  80

Asp Arg Gly Arg Gly Arg Ala Leu Pro Gly Gly Arg Leu Gly Gly Arg
                85                  90                  95

Gly Arg Gly Arg Ala Pro Glu Arg Val Gly Gly Arg Gly Arg Gly Arg
            100                 105                 110

Gly Thr Ala Ala Pro Arg Ala Ala Pro Ala Ala Arg Gly Ser Arg Pro
        115                 120                 125

Gly Pro Ala Gly Thr Met Ala Ala Gly Ser Ile Thr Thr Leu Pro Ala
    130                 135                 140

Leu Pro Glu Asp Gly Gly Ser Gly Ala Phe Pro Pro Gly His Phe Lys
145                 150                 155                 160

Asp Pro Lys Arg Leu Tyr Cys Lys Asn Gly Gly Phe Phe Leu Arg Ile

165                 170                 175

His Pro Asp Gly Arg Val Asp Gly Val Arg Glu Lys Ser Asp Pro His
            180                 185                 190

Ile Lys Leu Gln Leu Gln Ala Glu Glu Arg Gly Val Val Ser Ile Lys
        195                 200                 205

Gly Val Cys Ala Asn Arg Tyr Leu Ala Met Lys Glu Asp Gly Arg Leu
    210                 215                 220

Leu Ala Ser Lys Cys Val Thr Asp Glu Cys Phe Phe Phe Glu Arg Leu
225                 230                 235                 240

Glu Ser Asn Asn Tyr Asn Thr Tyr Arg Ser Arg Lys Tyr Thr Ser Trp
                245                 250                 255

Tyr Val Ala Leu Lys Arg Thr Gly Gln Tyr Lys Leu Gly Ser Lys Thr
            260                 265                 270

Gly Pro Gly Gln Lys Ala Ile Leu Phe Leu Pro Met Ser Ala Lys Ser
        275                 280                 285

<210>  3
<211>  129
<212>  DNA
<213>  Homo sapiens

<400>  3
tattctactg acttcggtgt gagttggaat tatctggtcc ctcagtgctt gcctgctgac      60

ccaaaatgct ctggaagtgt ttctcagcca tctgtattct ttccaactaa agggtggaaa     120

aggatcacc                                                             129

<210>  4
<211>  129
<212>  DNA
<213>  Homo sapiens

<400>  4
ataagatgac tgaagccaca ctcaacctta atagaccagg gagtcacgaa cggacgactg      60

ggttttacga gaccttcaca aagagtcggt agacataaga aaggttgatt tcccaccttt     120

tcctagtgg                                                             129

<210>  5
<211>  62
<212>  DNA
<213>  Homo sapiens

<400>  5

atctgtattc tttccaacta aagggtggaa aaggatcacc tacccacttc ctgaaagctt     60

ag                                                                  62

<210>  6
<211>  58
<212>  DNA
<213>  Homo sapiens

<400>  6
atctgtattc tttccaaagg gtggaaaagg atcacctacc cacttcctga aagcttag     58

<210>  7
<211>  48
<212>  DNA
<213>  Homo sapiens

<400>  7
atctgtaagg gtggaaaagg atcacctacc cacttcctga aagcttag               48

<210>  8
<211>  51
<212>  DNA
<213>  Homo sapiens

<400>  8
atctgtgaaa agggtggaaa aggatcacct acccacttcc tgaaagctta g            51

<210>  9
<211>  48
<212>  DNA
<213>  Homo sapiens

<400>  9
atctgtattc tttccaaagg atcacctacc cacttcctga aagcttag               48

<210>  10
<211>  57
<212>  DNA
<213>  Homo sapiens

<400>  10
atctgtattc tttccaaggg tggaaaagga tcacctaccc acttcctgaa agcttag      57